# EUROPEAN PATENT APPLICATION

(11) **EP 0 551 626 A1**
(43) Date of publication of application: **21.07.1993**
(21) Application number: 92121410.2
(22) Date of filing: 16.12.1992
(51) Int. Cl.: A61K 47/10, A61K 47/32, A61K 9/00

(54) **Thermoreversible gel as a liquid pharmaceutical carrier for a galenic formulation**

(30) Priority: 19.12.1991 YU 17/91
(71) Applicant: LEK, tovarna farmacevtskih in kemicnih izdelkov, d.d., SI-61107 Ljubljana (SI)
(72) Inventor: Kramaric, Anton, SL-61000 Ljubljana (SL); Resman, Aleksander, SL-61000 Ljubljana (SL); Kofler, Bojan, SL-64220 Skofja Loka (SL); Zmitek, Janko, SL-61000 Ljubljana (SL)
(74) Representative: Müller-Boré & Partner Patentanwälte

(57) **Abstract**

There is disclosed a thermoreversible gel as a liquid pharmaceutical carrier for a galenic formulation having improved thermo-rheological properties and with a gelling temperature interval between about 25°C and 37°C, which comprises
a) 10 to 30 wt.% of block copolymers of α-hydro-ω-hydroxypoly(oxyethylene)/poly(oxypropylene)/poly(oxy-ethylene) (Poloxamer) of the formula 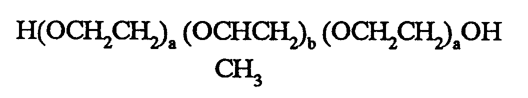 wherein a is at least 2 and b is at least 15 and the total portion of hydrophilic polyethylene units amounts to from 20 to 90 wt.% of the copolymer having a molecular weight from 1000 to above 16000;
b) 0.01 to 5 wt.% of carboxyvinyl polymer (carbomer) having a molecular weight from 1 x 10⁶ to 4 x 10 ⁶;
c) such an amount of a pharmaceutically acceptable base as necessary for adjusting the pH to a value between 4 and 8;
d) 20 to about 85 wt.% of water, and
e) optionally usual auxiliary agents.

There is also disclosed a liquid galenic formulation containing a therapeutically active amount of an active substance and a thermoreversible gel as a liquid pharmaceutical carrier, the active substance being selected from the group consisting of β-lactamic antibiotics and other antibacterial agents, chemotherapeutics, antiinfectives, topical anesthetics, anti-inflammatory and analgesic agents, antifungal agents, coronary vasodilators, antiviral agents, miotics and anticholinergics, mydriatic agents, antiglaucoma agents, antihistaminics, biogenic peptides and cosmetic agents.

At room temperature, such a formulation is in the sol form, which is converted into a gel form at a temperature about the body temperature and thus the application onto eye, skin and mucous membranes of the body cavities as well as the improved biopharmaceutical usefulness are made possible.

## Description

### Technical Field

(IPC A 61K 31/74, A 61K 31/78, A 61K 31/70)
The present invention belongs to the field of pharmaceutical industry and relates to a novel stable and improved thermoreversible gel as a liquid pharmaceutical carrier for a galenic formulation for application of active substances onto skin, eye or mucous membranes of body cavities.

### Technical Problem

There exists a constant need for providing a novel stable thermoreversible gel as a liquid pharmaceutical carrier for a galenic formulation for application of active substances onto skin, eye or mucous membranes of body cavities, which gel would make possible a simple and effective regulation of thermo-rheological properties of a novel carrier in the sol form as well as in the gel form, whereby the transition of a sol into a gel would be quick and complete with high gel viscosity (thick consistency) and thus with improved biopharmaceutical properties.

### Prior Art

Usually under the term gelling such a process is to be understood, which takes place at lowering the temperature. The concentrated polymer solutions usually have a very high viscosity due to the interaction of polymer chains leading to the formation of tridimensional structures in a solvent. Such viscous systems where polymers form a cross-linked reticular structure, into which structure a liquid phase is included, are referred to as gels. They are obtained by swelling a solid polymer or by reducing the solubility thereof in a solution. When forming a gel from the solution, each system is characterized by a critical gelling concentration, under which concentration no gel is formed. This concentration depends upon HLB (hydrophilic-lipophilic balance) of a polymer, upon the grade of the structure regularity, upon the interaction between the polymer and the solvent, upon molecular weight and upon the flexibility of polymer chains (cf. Florence A.T.D. Attwood, Physicochemical Principles of Pharmacy, Macmillan Press, 1989).

Another group of gels is also well-known, namely thermoreversible gels, which are characterized by gelling when heated and thus differ from the majority of polymer gels. The thermoreversible gelling makes possible a preparation of galenic forms, which are in the liquid sol form at temperatures under the body temperature, and when administered are converted into a semisolid gel form due to the warming to the body temperature. Also gels of block copolymers of α-hydro-ω-hydroxypoly(oxyethylene)ₐ/poly(oxypropylene)_{b}/poly(oxy-ethylene)ₐ with the generic name poloxamers or under trade mark Pluronic® belong to this group and represent a group of non-ionic surfactants of the formula 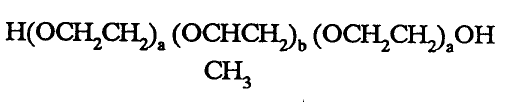
wherein a is an integer from 2 to 130 and b an integer from 15 to 67 and a total portion of polyethylene units amounts to from 20 to 90 wt.% of copolymer having molecular weight from 1000 to above 16000 as stated e.g. in The Merck Index, 11th Edition (1989) under monographic number 7537.

In comparison with other surfactants, which usually have an unchangeable hydrofobic moiety of the molecule and thus the changes in the properties thereof may be achieved only by modifying a hydrophilic moiety, in poloxamers both moieties of the molecule as well as the overall molecular weight may be modified. As evident from the above formula, the poloxamer molecule is built of the central lipophilic moiety (polyoxypropylene), which is surrounded from both sides by hydrophilic blocks of polyoxyethylene. Poloxamers are prepared by condensing propylene oxide with propylene glycol followed by condensing ethylene oxide on both ends of the polyoxypropylenic block. By modifying the chain lengths of polyoxyethylenic and polyoxypropylenic moieties of the molecule, different kinds of poloxamers are formed which differ from one another as to physical, physico-chemical and other properties. Thus e.g. poloxamer 407 in accordance with INN nomenclature (International Non-Proprietary Name) has the commercial name Pluronic F127.

Poloxamers have become interesting because of their characteristic of thermoreversible gelation, whereat aqueous dispersions of poloxamers are converted upon heating from the sol form into a gel form as disclosed by I.R. Shmolka, J. Biomed. Mat. Res. 6, 571-582 (1972), US patent 3,740,421. Therein a galenic form is described, which before or during the application (at room temperature) is liquid and upon heating to body temperature is converted into a semi-solid gel form.

CA patent 1,072,413 discloses a preparation of thermoreversible gels of different kinds of poloxamers, which also gelatinize at higher temperatures, and of gels having high Pluronic F127 concentration, which, due to the effect of additives, gelatinize at a temperature above 26°C. A gel-forming composition comprising from 15 to 50 wt.% of poloxamer, from 0 to 25 wt.% by weight of an additive which modifies the gel properties and contains more than one polymeric block made up of ethylene oxide, propylene oxide or combination thereof, such as Tetronic® or Tergitol®, and from 85 to 50 wt.% of a compatible aqueous phase is disclosed, which composition is liquid at room temperature and forms a gel upon warming to a temperature above 26 °C. While the gelling temperatures are exactly defined therein, said patent does not disclose gel viscosities at the body temperature.

US patent 4,188,373 discloses a thermoreversible gel on the basis of poloxamers (Pluronic), wherein the desired gelling temperature (sol-gel transition temperature) is regulated by modifying the polymer concentration, namely the lower the polymer concentration the higher the temperature. The possibility to obtain a gel having the desired consistency by using commercially available Pluronics while maintaining the desired gelling temperature in a physiologically acceptable temperature range between 26 °C and 35 °C, is limited.

The use of Tetronic® products (BASF Wyandotte Corporation) for preparing thermoreversible gels and regulating the thermo-rheological parameters by the concentration of these polymers, the pH and the ionic strength of the additives is disclosed in US patents 4,474,751, 4,474,753 and 4,478,822, wherefrom therapeutical advantages of the thermoreversible gel over a usual gel are evident.

US patent 4,803,066 discloses a combination of silver sulphadiazine with an antimicrobial azole active substance such as clotrimazole or metronidazole for a topical application onto skin. As preferable substrates there are mentioned Pluronic gels of a molecular weight between 4600 and 13500 and a concentration from 20 to 50%.

Pluronic F127 gels may, in addition to having specific thermo-rheological properties, also be effective in dermal absorption of the active substance. In J. Pharm. Sci, 79, 11, (1990) there is disclosed a comparison of the anti-inflammatory activity of 1% ketoprofen concentration in 20% Pluronic gel F127 with the ketoprofen activity in Carbopol gel.

Handbook of Pharmaceutical Excipients, Am. Pharm. Association (1986) teaches that carbomers (non-proprietary name, chemical name carboxypolymethylene; CAS Registry Number 9007-20-9), e.g. Carbopol (B.F. Goodrich Chemical Co.) are synthetic cross-linked polymers of acrylic acid, copolymerized with about 0.75 to 2 wt.% of polyalkylsucrose, of a high molecualar weight (from about 1 x 10⁶ to 4 x 10⁶). The carboxyl group portion in the end product amounts to 56 to 68 wt.%. E.g. Carbomer 934P (Carbopop 934) has a molecular weight of 3 x 10⁶.

In G.Dumortier et al., Thermorheological behaviour of Poloxamer 407 solutions: Application to the determination of the transition temperature and of the yield stress. Procc. from 10th Pharmaceutical Technology Conference, Bologna 1991, the effect of Pluronic F127 on the gelling temperature is disclosed and it is ascertained that gels having lower Pluronic F127 concentration exhibit a less drastical sol-gel transition in comparison with more concentrated ones.

The effects of particular additives for regulating gel viscosity and gelling temperature on thermo-rheological properties of poloxamer gels are also disclosed in the literature.

Specific interactions of poloxamers and polyacrylic acid - i.e. interfacial complexing between polyacrylic acid and different poloxamers included as surfactants - were disclosed in connection with stabilization of multiple emulsions by M.L. Cole et al., J. Pharm.Pharmacol. 42, 136P (1990). It was found that the addition of small amounts of poloxamers to aqueous dispersions of polyacrylic acid results in turbidity and/or precipitate formation. Upon further addition of poloxamer, the precipitate is dissolved and a clear solution is formed.

From the patent literature and other literature, disadvantages of the prior art solutions and ways of regulating thermo-rheological properties of aqueous poloxamer dispersions are well-known. Elevating the poloxamer concentration results in an essential increase of the gel viscosity at the body temperature, however, the gelling temperature is lowered thereby and such galenic form may gelatinize already under the temperature of the application. Such a form must be thus applied in the form of gel and in this way all its advantages over a standard gel as a medicinal form disappear. While the means for increasing the gelling temperature such as the addition of a poloxamer having a lower molecular weight and a higher content of the hydrophilic moiety of the molecule are well-known, the temperature range of gelling is essentially increased in such cases and thus it is impossible to achieve correspondingly high differences in the viscosity of the formulations before or during the application and afterwards.

So far these problems have been solved best as disclosed in the US patent 4,474,753, wherein the gelling temperatures are varied by means of pH values, supposedly without significant changes in gel consistency. The solution as disclosed has some advantages, yet due to the galenic form it is necessary that the pH value is in the physiologically acceptable range where said effects are already substantially smaller.

Since an effective increase in the gel viscosity (consistency) in the prior art solutions may only be provided by increasing the poloxamer concentration, in which case the gelling temperature of the formulation is decreased, only a polymer concentration just providing for the gelling temperature above 25 °C may be used. This also represents a serious, hitherto unsolved limitation in achieving higher gel viscosities, which is the principal object in preparing formulations for application onto skin, eye or mucous membranes, where in addition to a simple and painless application made possible by a low viscosity, also an appropriate protection against mechanical and other influences should be attained by replacing the damaged surface by the formulation.

### The Inventive Solution

The object of the present invention was to prepare a novel stable and improved thermoreversible gel as a liquid pharmaceutical carrier for application of active substances onto skin, eye or mucous membranes of body cavities, having improved themo-rheological and therapeutical properties and lacking the prior art disadvantages.

It has been surprisingly found that by adding 0.01% to 5% of a carbomer to 10 to 30 wt.% aqueous poloxamer solutions a reduction of sol viscosity at temperatures under the gelling temperatures and a high increase of gel viscosity at room temperature and, in addition, also a quicker transition from sol to gel are achieved. In this way the physico-chemical characteristics of a thermoreversible gel in the range of physiological applicability as well as at a pH value between 4 and 9 may be regulated effectively and rapidly.

Due to the reduced sol viscosity the topical application is facilitated, the coverage of the damaged site is improved and, due to the larger contact surface, the release of the active substance is improved, the result being improved bio-availability.

A substantiallly higher viscosity and a thicker consistency of the gel at the body temperature lead to therapeutical advantages which relate (in addition to the disclosed ones) especially to a better protection of the application site against outside effects and to the help in maintaining constancy of the interior, thus supporting a quicker and more successful treatment.

Because of the contemplated use of a galenic form for application onto skin, eye or mucous membranes of body cavities, it is desirable that a thermoreversible gel has the highest possible viscosity in the range of 32 °C to 37 °C, additionally a quick transition from sol to gel in the narrowest posible temperature interval is desirable.

These objects were achieved simply and effectively by a novel thermoreversible gel of the invention with the regulation of thermo-rheological properties of a novel carrier in the sol form as well as in the gel form within a narrow gelling temperature interval.

In order to find the best possible form of a liquid pharmaceutical carrier for a galenic formulation according to the invention, the effect of poloxamer concentration (Pluronic F127), of carbomer concentration (Carbopol 934P) and of neutralisation grade of acrylic acid in the carbomer (Carbopol 934P) on thermo-rheological properties of aqueous poloxamer dispersions as well as the effect of carbomer concentration on the temperature-dependent rate of transition from sol to gel were determined.

The effect of Pluronic F127 concentration on thermo-rheological properties of gels is evident from the Diagram 1. By increasing the Pluronic F127 concentration, the maximum viscosity is increased. The optimum Pluronic F127 concentration in a dermal galenic formulation gelling at the temperature above room temperature and having the maximum viscosity at the body temperature is between 15 and 20%.

The effect of addition of some polymers on the thermo-rheological properties of 20% Pluronic F127 gels having a composition as given in Table I is evident from the Diagram 2. The addition of 0.5 wt.% Carbopol 934P to a 20% aqueous Pluronic F127 dispersion results in a maximum viscosity at a minimum reduction of the sol-gel transition temperature.

The compositions of individual aqueous dispersions, including dispersions according to the invention, and thermo-rheological characteristics thereof are given in Tables I, II, III and IV.

**TABLE I**

| The effect of some polymers on important thermo-rheological parameters of their dispersions | | | | | | |
|---|---|---|---|---|---|---|
| | A1 | A7 | A10 | A11 | A17 | A24 |
| Pluronic F 127 | 20.0% | 20.0% | 20.0% | 20.0% | - | 25.00% |
| Methocel E4 | - | 0.5% | - | - | - | - |
| xanthan gum | - | - | 0.5% | - | - | - |
| Carbopol 934P | - | - | - | 0.5% | - | - |
| NaOH | - | - | - | 0.207% | - | - |
| polyethylene glycol 6000 | - | - | - | - | 10% | - |
| ηmax. (Pa.s) | 6.99 | 7.817 | 9.059 | 19.94 | no | 12.96 |
| T (ηmax.) (°C) | 39.7 | 39.9 | 57.2 | 38.1 | gelling | 39.6 |

**TABLE II**

| The effect of adding Carbopol 934P on important thermo-rheological parameters of 20% Pluronic F 127 gels | | | | |
|---|---|---|---|---|
| | A1A | A32 | A33 | A11 |
| Pluronic F 127 | 20.0% | 20.0% | 20.0% | 20.0% |
| Carbopol 934P | - | 0.1% | 0.3% | 0.5% |
| NaOH | - | 0.035% | 0.111% | 0.207% |
| pH | | 6.83 | 6.79 | 6.95 |
| ηmax. (Pa.s) | 7.07 | 9.44 | 15.56 | 19.94 |
| T(ηmax.) (°C) | 38.5 | 39.3 | 39.65 | 38.1 |

**TABLE III**

| The effect of adding Carbopol 934P on important thermo-rheological parameters of 15% Pluronic F 127 gels | | | | |
|---|---|---|---|---|
| | A25 | A35 | A39 | A34 |
| Pluronic F 127 | 15.0 % | 15.0% | 15.0% | 15.0% |
| Carbopol 934P | - | 0.1% | 0.3% | 0.5% |
| NaOH | - | 0.04% | 0.11% | 0.197% |
| pH | | 7.02 | 6.91 | 6.81 |
| η max. (Pa.s) | 0.04 | 1.678 | 3.996 | 4.471 |
| T (η max.) (°C) | 39.4 | 38.9 | 37.5 | 36.1 |

**TABLE IV**

| The effect of adding Carbopol 934P on important thermo-rheological parameters of 18% Pluronic F 127 gels | | |
|---|---|---|
| | A37 | A38 |
| Pluronic F 127 | 18.0% | 18.0% |
| Carbopol 934P | 0.3% | 0.5% |
| NaOH | 0.111% | 0.197% |
| NaCl | - | - |
| pH | 6.84 | 6.83 |
| η max. (Pa.s) | 11.91 | 11.84 |
| T (η max.) (°C) | 42.9 | 40.2 |

The differences are also very clearly evident from thermorheograms (Diagrams 3 and 4) where the viscosity of the sample A11 is lower at lower temperatures than the viscosity of 0.5% Carbopol 934P gel (sample A11 P) in spite of the fact that, because of the addition of Pluronic F127 in the sample A11, the actual portion of Carbopol 934P in water is higher.

As evident from the Diagram 5, the combination of Carbopol 934P and Pluronic F127 results in a sinergistic increase of gel viscosity since the gel viscosity is much higher than the sum of viscosities of the individual gelling components and is a result of specific physico-chemical interactions between a carbomer and a poloxamer. In this way a simple and effective regulation of the thermo-rheological properties of such carrier in the sol state as well as in the gel state is attained, achieving a reduction of sol viscosity, an increase of gel viscosity and a rapid transition from sol to gel, i.e. a narrow gelling temperature interval. Thus the preparation of the aqueous galenic form according to the invention is made possible, which form gives essentially better results than the forms hitherto described in the literature. The carbomer alone has no essential effect on gelling temperature and is thus different from other viscosity regulators, which with the increase of gel viscosity at body temperture decrease the gelling temperature as a rule.

The effect of neutralisation grade of a carbomer with basic substances on thermo-rheological properties of aqueous poloxamer dispersions is evident from the Diagram 6. With the increase of neutralisation grade of a carbomer, the gel viscosity increases and the gelling temperature decreases. The effect of interactions causing the increase of gel viscosities is maximal at the 0.3% carbomer concentration. A further increase of carbomer concentration has a smaller effect on the viscosity than the contribution of the carbomer gel alone. In this case probably a partial solubilization of acrylic acid polymers is in question, resulting in the decrease of the gelling ability.

It has been found that, irrespective of the poloxamer concentration, galenic forms containing 0.3% of the carbomer are gelatinized most rapidly.

In the literature, so far two processes of colloidal dissolution of poloxamers in water (Pharm.Acta Helv. 65(4), 1990, 105-106) have been disclosed, namely a cold process at a temperature under 6°C and a hot process at a temperature above 60°C. Since both processes are unsuitable for industrial use (the first process requires a special cooling system in reactors, giving rise to long preparation times and to energy losses, whereas the second process is unfavourable because the sol is converted upon cooling to room temperature into a cooled sol form *via* an intermediate gel and thus at higher viscosity the overloading of the stirring system may take place), a novel process, not yet disclosed in the literature, has been developed, which is advantageous in that the gel is prepared at room temperature resulting in energy saving and in shortened preparation time. Therefore also this novel process is one of the objects of the invention. It was found out by testing that thermo-rheological properties of thermoreversible gel samples were exactly the same irrespective of the process - either prior art process or the present process - for the preparation thereof.

According to the present invention, a carbomer (Carbopol 934P) is homogenously dispersed in demineralized water at room temperature and the pH is adjusted with an aqueous alkali solution such as sodium hydroxide solution to a value between 4 and 8. Into the clear colloidal dispersion obtained, a poloxamer (Pluronic F127) is blended and left to swell for 1 hour. The dispersion thus prepared is homogenized in an appropriate stirring device. Separately, a homogenous dispersion of the active substance in a small amount of water is prepared and blended into the obtained pharmaceutical carrier to give a galenic form, which is liquid at a temperature about room temperature or thereunder, and by all means under 25°C.

The properties of the thermoreversible gel such as sol form at the time of application, a thermally dependent gelling after application with a relatively high viscosity and a thick gel consistency achieved, the lack of toxicity, the absence of allergic reactions, and the hydrophility with the possibility of regulating the permeability for water vapours and skin secretions, make possible a broad use of said gel as a pharmaceutical carrier for different active substances and as a protective formulation for therapeutic purposes, which excells in a simple and painless application (in a sol state), protects the site of application against the outside (skin substitute function), may be readily removed (washing with water) and is able to solubilize active substances.

Thermoreversible gels are useful in the application onto eye, skin and mucous membranes of body cavities, such as nose, rectum, vagina, urethra or buccal part in the mouth.

The galenic form is applied onto eye in the same was as eye drops, whereas after sol to gel transition, which occurs because of the eye temperature, the galenic form behaves on the application site as an ointment gel.

In such a way there is a benefit from the advantages of eye drops such as simple application, good and uniform distribution over the eyeball, good acceptance in patients etc. as well as from the advantages of eye ointments such as a great improvement of bio-availability in comparison with eye drops, prolongation of the effect, reduced frequency of application etc.

In applying the galenic form according to the invention onto mucous membranes, the advantages are achieved in comparison with the usual gel or ointment, which advantages are a better contact with the mucous membrane and a better coating thereof, providing better therapeutical effects in systemic as well as in topical application of the active substances. The galenic form of the invention is distinctly advantageous over commonly used galenic forms when applied onto a damaged skin as a result of burns, mechanical lesions etc. The application thereof is painless and simple because it is applied in the sol form or by spraying. Alter application the formulation gelatinizes because of the higher skin temperature. The gel as formed having a relatively thick consisteny may serve as a substitute for the damaged skin, i.e. as a protection of the interior against the outside. Lipophilic, i.e. hydrophobic additives may affect the permeability for water vapours and thus affect the removal of possible skin secretions as well as hydratation of inside layers. These are factors which substantially affect a successful healing and later scarring. Since the gel is hydrophilic by nature, its elimination by washing with water is simple, especially if the water temperature is lower than the gelling temperature. Due to the gelling on the application site, also the fixation of the dressing material on the wound is better.

For the application onto eye, skin or mucous membranes of the body cavities each active substance compatible with the ingredients of the thermoreversible gel in the pharmaceutical carrier is useful.

Examples of the active substances that may be included into a pharmaceutical carrier according to the invention are as follows:
1.
   β-lactamic antibiotics and other antibacterial agents, such as ampicillin, amoxicillin, cefoxitin, cephalexin, cephalotin sodium, ceftizoxime sodium, cefuroxime axetil, cefaclor, ceftriaxone disodium, cefotaxime sodium, vancomycin, lincomycin, clindamycin and clindamycin-2-phosphate, doxycycline, oxytetracycline, N-form-imidoil thienamycin and other tienamycin derivatives.
2.
   Chemotherapeutics or antiinfectives, such as silver sulfadiazine, pipemidic acid, norfloxacin, perfloxacin, ciprofloxacin, enrofloxacin, lomefloxacin, fleroxacin, ofloxacin, iodine, chloramines, benzalkonium chloride.
3.
   Topical anesthetics, such as lidocaine, bupivacaine, mepivacaine, cocaine, procaine.
4.
   Anti-inflammatory and analgesic agents, such as indomethacin, ketoprofen, ibuprofen, ibuproxam, ketorolac, sulindac, nabumetone, etodolac, flurbiprofen, diclofenac sodium, piroxicam, betamethasone, dexamethasone, morphine, buprenorphine, diflunisal, acetylsalicylic acid.
5.
   Antifungal agents, such as econazole nitrate, miconazole nitrate, clotrimazole, nystatin, ketoconazole.
6.
   Coronary vasodilators, such as isosorbide mono- or dinitrate, nifedipine, nicardipine hydrochloride, nimodipine, nitrendipine, felodipine, amlodipine besilate, isradipine and nicorandil.
7.
   Antiviral agents, such as acyclovir, ganciclovir, idoxuridine, interferon, adenosine arabinoside (Ara-A).
8.
   Miotics and anticholinergics, such as pilocarpine, epinephrine, physostigmine, neostigmine.
9.
   Mydriatic agents, such as atropine, scopolamine, ephedrine.
10.
   Antiglaucoma agents, such as timolol maleate, R-timolol and a combination of timolol or R-timolol with pilocarpine.
11.
   Antihistaminics, such as disodium cromoglycate, oxatomide, nedocromil, azelastine hydrochloride and ketotifen fumarate.
12.
   Biogenic peptides, such as calcitonin, insulin, elcatonin, urokinase, TNF (Tumor Necrosis Factor), erythropoietin, interferons, tPA (Tissue Plasminogen Activator), interleukins (1,2,3,4) such as IL-2.
13.
   Cosmetic agents, such as benzoyl peroxide, minoxidil.
14.
   Protective agents against damages caused by the action of UV irradiation.
15.
   Anaesthetics for catheterization or cystoscopy in urological examination.

In microbiological tests for asserting the activity of preservatives according to the method USP XXII, the inventive thermoreversible gel alone - without active substance - has a very good antimicrobial action and to the inventive galenic form no preservatives need not be added and thus several undesired effects are avoided such as allergies, hypersensitivity, irritation etc., which effects usually appear when incorporating preservatives like antimicrobial agents into galenic forms. These unfavourable effects manifest themselves especially in cases of damaged cornea. Thus the galenic form according to the invention is essentially advantageous over usual aqueous galenic forms and represents a valuable enrichment of the Art.

Optionally, the galenic form may contain usual auxiliary agents such as propylene glycol, glycerol, various native and synthetic triglycerides (peanut oil, Cetiol® etc.) etc.

The invention is illustrated with non-limiting Examples and from the thermograms thereof there is evident a high vicosity of the thermoreversible gel containing a compatible active substance and a poloxamer and carbomer as carrier ingredients.

### Example 1

### Liquid thermoreversible galenic formulation of betamethasone-17,21-dipropionate

| Composition | wt. % |
|---|---|
| betamethasone-17,21-dipropionate | 0.05 |
| Pluronic F127 | 18.0 |
| Carbopol 934P | 0.3 |
| sodium hydroxide, 10% aqueous solution | 5 |
| demineralized water | ad 100 |

In demineralized water (75 weight parts) Carbopol 934P (0.3 wt. part) was homogenously dispersed at room temperature and the pH value was adjusted to 6 to 7 with a 10% aqueous sodium hydroxide solution (5 wt. parts). Pluronic F127 (18.0 wt. parts) was blended into the clear colloidal dispersion and left to swell for 1 hour. The carrier obtained was stirred with a propeller stirrer at 2250 rpm for 5 minutes. Separately, a homogenous dispersion of betamethasone dipropionate (0.05 wt. parts) in the remaining water (about 1.2 wt. parts) was prepared and blended into the carrier in the sol form. A liquid galenic formulation was obtained at a temperature of about room temperature, but not above 25°C.

At the application, the galenic formulation was converted, when heated to the body temperature, from the liquid sol form into a semi-solid gel form, from which the active substance was released at the site of the application.

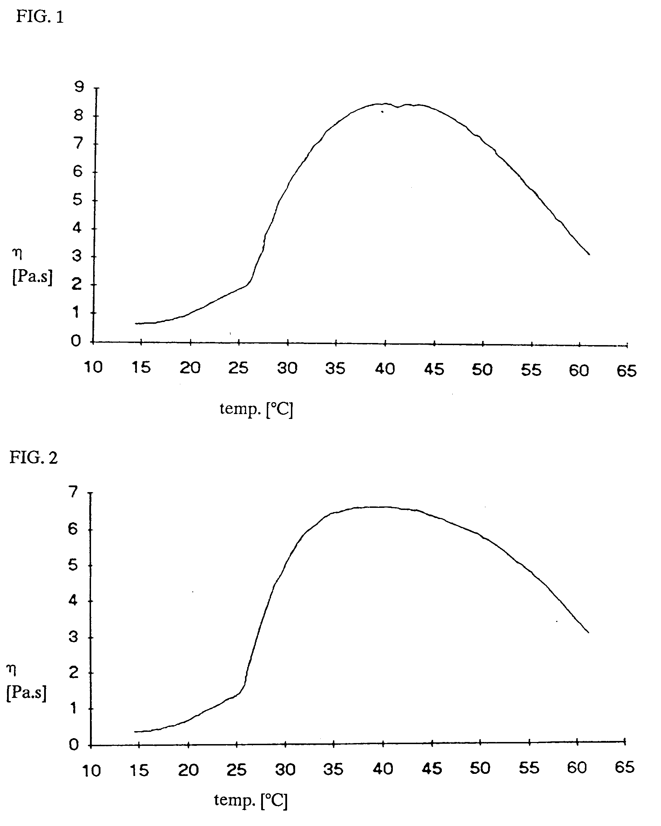

The thermogram of said galenic formulation is shown in Fig.1.

### Example 2

### Liquid thermoreversible galenic formulation of dexamethasone-21-phosphate disodium salt

| Composition | wt.% |
|---|---|
| dexamethasone-21-phosphate disodium salt | 0.1 |
| Pluronic F127 | 18.0 |
| Carbopol 934P | 0.3 |
| sodium hydroxide, 10% aqueous solution | 5.0 |
| demineralized water | ad 100.0 |

It was proceeded as in Example 1 with the exception that as the active substance dexamethasone-21-phosphate disodium salt (0.1 wt.%) was used.

The thermogram of said galenic formulation is shown in Fig.2.

### Example 3

### Liquid thermoreversible galenic formulation of silver sulfadiazine

| Composition | wt. % |
|---|---|
| silver sulfadiazine | 1.0 |
| Pluronic F127 | 18.0 |
| Carbopol 934P | 0.3 |
| sodium hydroxide, 10% aqueous solution | 5.0 |
| demineralized water | ad 100.0 |

It was proceeded as in Example 1 with the exception that as the active substance silver sulfadiazine (1.0 wt.%) was used.

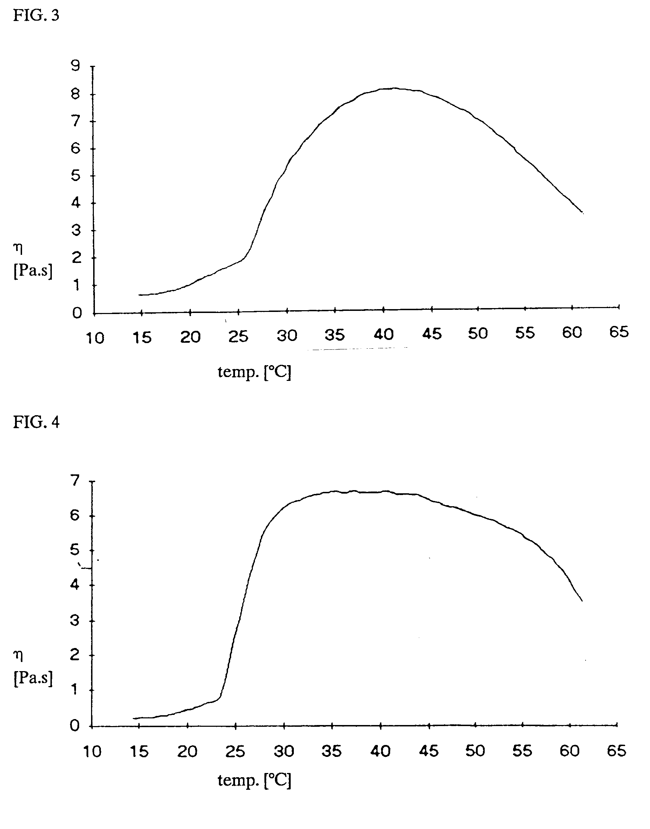

The thermogram of said galenic formulation is shown in Fig.3.

### Example 4

### Liquid thermoreversible galenic formulation of econazole nitrate

| Composition | wt. % |
|---|---|
| econazole nitrate | 1.0 |
| Pluronic F127 | 18.0 |
| Carbopol 934P | 0.3 |
| sodium hydroxide, 10 % aqueous solution | 5.0 |
| demineralized water | ad 100.0 |

It was proceeded as in Example 1 with the exception that as the active substance econazole nitrate (1.0 wt.%) was used.

The thermogram of said galenic formulation is shown in Fig.4.

### Example 5

### Liquid thermoreversible galenic acyclovir formulation

| Composition | wt.% |
|---|---|
| acyclovir | 5.0 |
| Pluronic F127 | 18.0 |
| Carbopol 934P | 0.3 |
| sodium hydroxide, 10% aqueous solution | 5.0 |
| demineralized water | ad 100.0 |

It was proceeded as in Example 1 with the exception that as the active substance acyclovir (5.0 wt.%) was used.

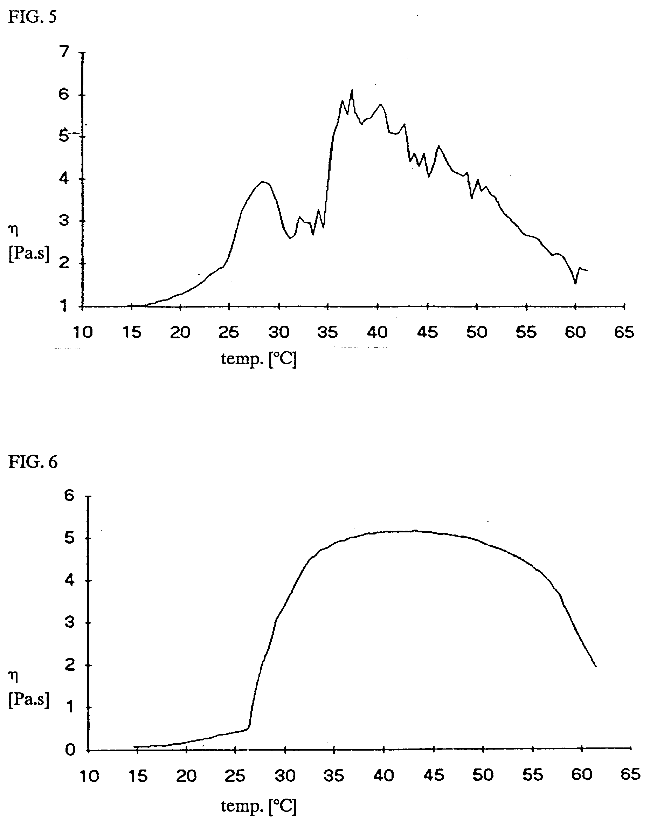

The thermogram of said galenic formulation is shown in Fig.5.

### Example 6

### Liquid thermoreversible galenic formulation of lidocaine hydrochloride

| Composition | wt. % |
|---|---|
| lidocaine hydrochloride | 2.0 |
| Pluronic F127 | 18.0 |
| Carbopol 934P | 0.3 |
| sodium hxdroxide, 10 % aqueous solution | 5.0 |
| demineralized water | ad 100.0 |

It was proceeded as in Example 1 with the exception that as the active substance lidocaine hydrochloride (2.0 wt.%) was used.

The thermogram of said galenic formulation is shown in Fig.6.

### Example 7

### Liquid thermoreversible galenic ketoprofen formulation

| Composition | wt. % |
|---|---|
| ketoprofen | 5.0 |
| Pluronic F127 | 18.0 |
| Carbopol 934P | 0.3 |
| sodium hydroxide, 10% aqueous solution | 5.0 |
| demineralized water | ad 100.0 |

It was proceeded as in Example 1 with the exception that as the active substance ketoprofen (5.0 wt.%) was used.

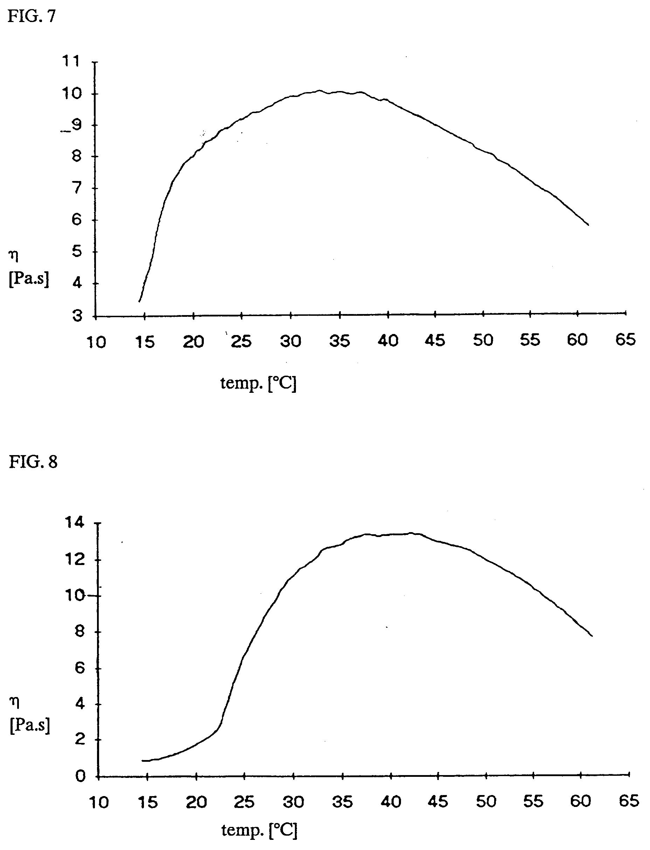

The thermogram of said galenic formulation is shown in Fig.7.

### Example 8

### Liquid thermoreversible galenic formulation of benzoyl peroxide

| Composition | wt. % |
|---|---|
| benzoyl peroxide | 5.0 |
| Pluronic F127 | 18.0 |
| Carbopol 934P | 0.3 |
| sodium hydroxide, 10% aqueous solution | 5.0 |
| demineralized water | ad 100.0 |

It was proceeded as in Example 1 with the exception that as the active substance benzoyl peroxide (5.0 wt.%) was used.

The thermogram of said galenic formulation is shown in Fig.8.

### Comparative Example

It was tried to prepare galenic formulations of some antibiotics using the same carrier as in the previous Examples.

| Composition | wt. % |
|---|---|
| chloramphenicol palmitate | 5.0 |
| Pluronic F127 | 18.0 |
| Carbopol 934P | 0.3 |
| sodium hydroxide, 10% aqueous solution | 5.0 |
| demineralized water | ad 100.0 |

It was proceeded as in Example 1 with the exception that as the active substance chloramphenicol palmitate (5.0 wt.%) was used.

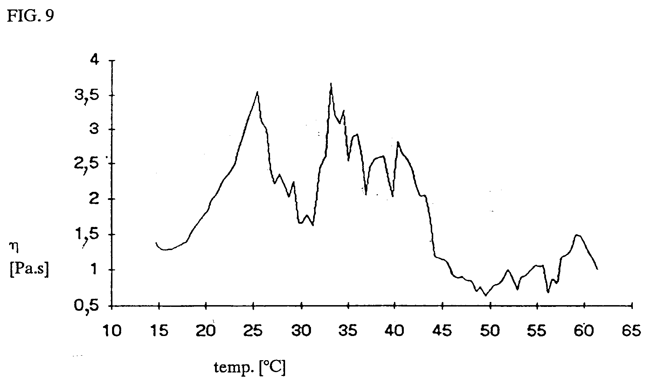

The thermogram of the formulation obtained is shown in Fig.9.

From the irregular thermogram of the chloramphenicol palmitate formulation it is evident the incompatibility thereof with the thermoreversible carrier.

Also gentamycin sulfate, bacitracin and neomycin sulfate show similar incompatibilities with the thermoreversible carrier.

### Microbiological test

In the microbiological test of assesing preservative activities according to the method of USP XXII, p.1478, it was found that the thermoreversible galenic formulation according to the present invention itself exhibits a sufficient antimicrobial action and thus the addition of preservatives is not necessary as evident from Table V.

**Table V**

| test microorganism (TM) acc. to USP XXII | concentration of test microorganism | | | | | |
|---|---|---|---|---|---|---|
| | start. conc. TM | start. conc. sample | day 7 | day 14 | day 21 | day 28 |
| Staphylococcus aureus | 10⁶/g | >3.10⁵ | 5.10⁴ | 0 | 0 | 0 |
| Pseudomonas aeruginosa | 10⁶/g | 0 | 0 | 0 | 0 | 0 |
| Escherichia coli | 10⁶/g | >10⁶ | 5.10⁵ | 6.10³ | 10³ | 10³ |
| Candida albicans | 10⁶/g | >10⁶ | 10⁴ | >10⁵ | 10⁴ | 5.10³ |
| Aspergillus niger | 10⁶/g | 3.10⁴ | 10² | >10² | >10² | >5.10² |

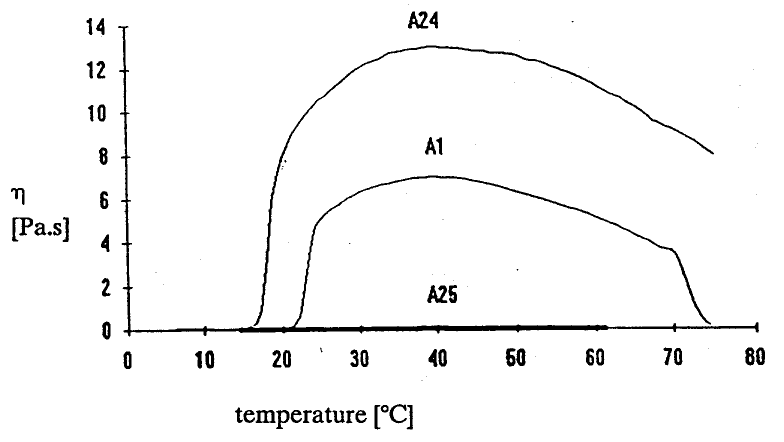

### Diagram 1:

The comparison of gel thermorheograms with various Pluronic F 127 concentrations

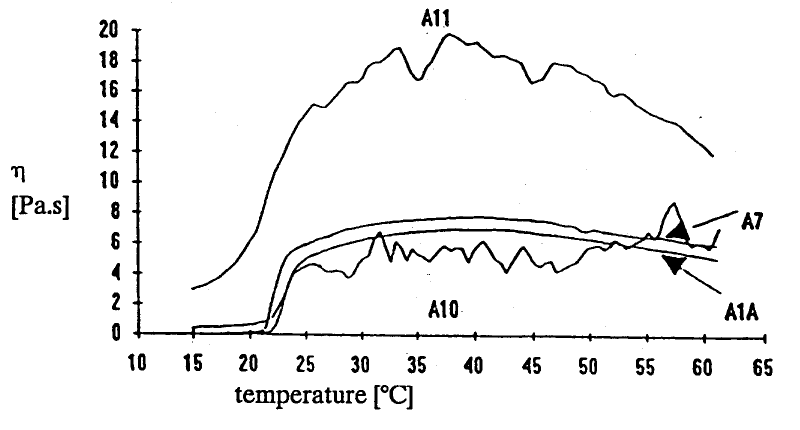

### Diagram 2:

The effect of addition of some polymers on thermorheological properties of 20% Pluronic F 127 gels

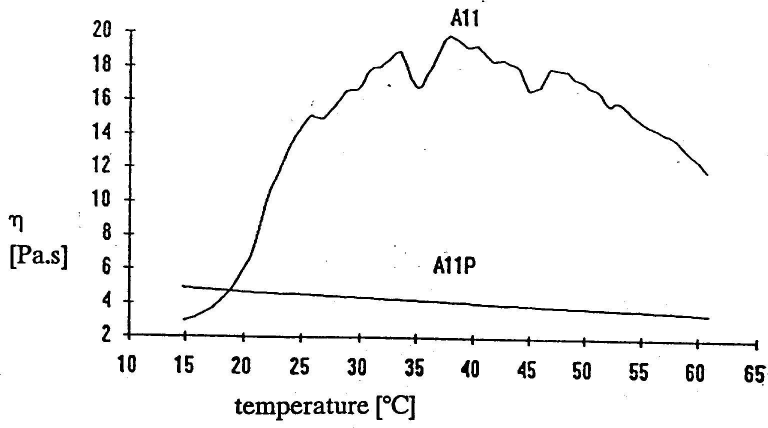

### Diagram 3:

The comparison of viscosities of 0.5 % Carbopol 934P (A11P) gel and a sample of 20 % Pluronic F 127 gel with the addition of 0.5 % Carbopol 934P (A11).

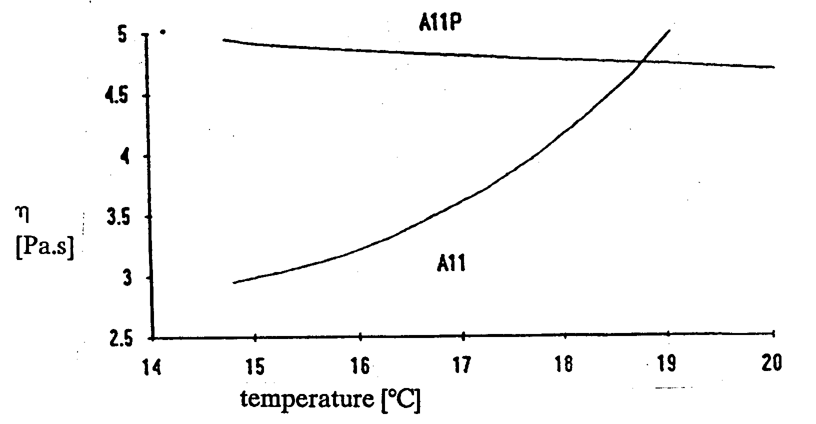

### Diagram 4:

The comparison of viscosities of 0.5 % Carbopol 934P (A11P) gel and a sample of 20 % Pluronic F 127 gel with the addition of 0.5 % Carbopol 934P (A11).

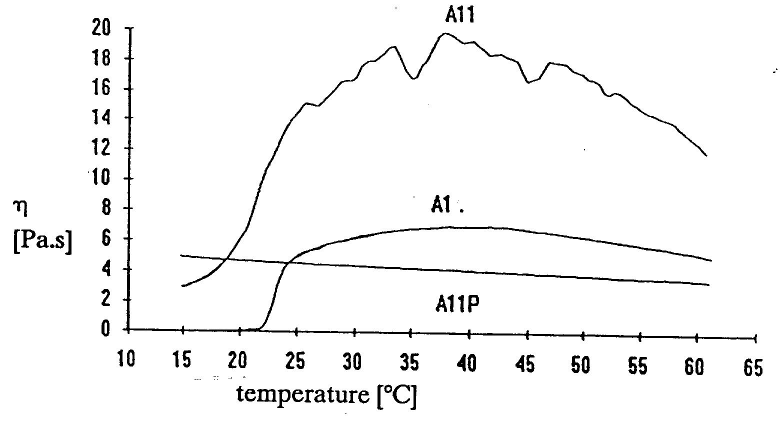

### Diagram 5:

The comparison of viscosities of 0.5 % Carbopol 934P (A11P) gel and of 20% Pluronic F 127 gel and of 20% Pluronic F 127 gel with the addition of 0.5 % Carbopol 934P (A11).

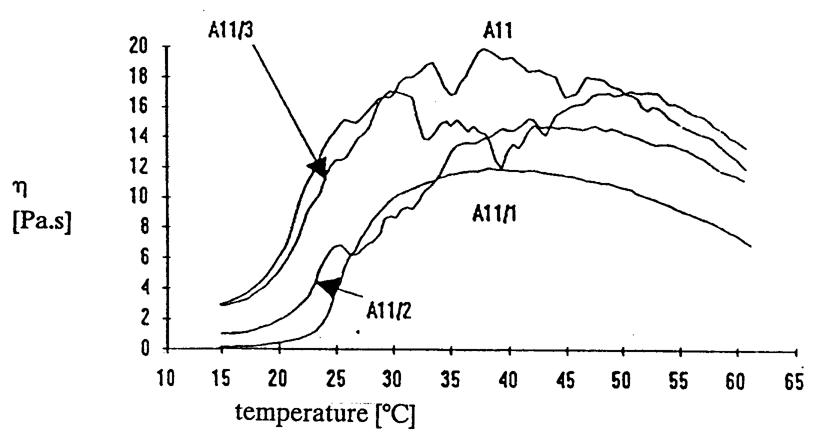

### Diagram 6:

The effect of the neutralization grade of acrylic acid with sodium hydroxide on thermo-rheological properties of 20 % Pluronic F 127 gels with the addition of 0.5 % Carbopol 934P (A11)/1 (pH 4.16), A11/2 (pH = 4.91), A11/3 (pH = 6.05), A11 (pH = 6.95)

## Claims

1. Thermoreversible gel as a liquid pharmaceutical carrier for a galenic formulation having improved thermo-rheological properties and with a gelling temperature interval between about 25°C and 37°C, characterized in that it comprises
a) 10 to 30 wt.% of block copolymers of α-hydro-ω-hydroxypoly(oxyethylene)/poly(oxypropylene)/poly(oxy-ethylene) (Poloxamer) of the formula 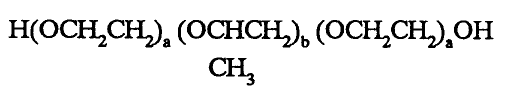 wherein a is at least 2 and b is at least 15 and the total portion of hydrophilic polyethylene units amounts to from 20 to 90 wt.% of the copolymer having a molecular weight from 1000 to above 16000;
b) 0.01 to 5 wt.% of carboxyvinyl polymer (carbomer) having a molecular weight from 1 x 10⁶ to 4 x 10⁶;
c) such an amount of a pharmaceutically acceptable base as necessary for adjusting the pH to a value between 4 and 8;
d) 20 to about 85 wt% water and
e) optionally usual auxiliary agents.

2. Liquid galenic formulation characterized in that it contains a therapeutically active amount of the active substance and a thermoreversible gel according to claim 1 as a liquid pharmaceutical carrier.

3. Liquid galenic formulation according to claim 2, characterized in that the active substance is selected from a group consisting of β-lactamic antibiotics and other antibacterial agents, chemotherapeutics, antiinfectives, topical anesthetics, antiinflammatory and analgesic agents, antifungal agents, coronary vasodilators, antiviral agents, miotics and anticholinergics, mydriatic agents, antiglaucoma agents, antihistaminics, biogenic peptides and cosmetic agents.

4. Liquid galenic formulation according to claim 2, characterized in that β-lactamic antibiotics and other antibacterial agents are selected from the group consisting of ampicillin, amoxicillin, cefoxitin, cephalexin, cephalotin sodium, ceftizoxime sodium, cefuroxime axetil, cefaclor, ceftriaxone disodium, cefotaxime sodium, vancomycin, lincomycin, clindamycin and clindamycin-2-phosphate, doxycycline, oxytetracycline, N-form-imidoil thienamycin and other tienamycin derivatives.

5. Liquid galenic formulation according to claim 2, characterized in that chemotherapeutics or antiinfectives are selected from a group consisting of silver sulfadiazine, pipemidic acid, norfloxacin, perfloxacin, ciprofloxacin, enrofloxacin, lomefloxacin, fleroxacin, ofloxacin, iodine, chloramines and benzalkonium chloride.

6. Liquid galenic formulation according to claim 2, characterized in that topical anesthetics are selected from a group consisting of lidocaine, bupivacaine, mepivacaine, cocaine and procaine.

7. Liquid galenic formulation according to claim 2, characterized in that antiinflammatory and analgesic agents are selected from a group consisting of indomethacin, ketoprofen, ibuprofen, ibuproxam, ketorolac, sulindac, nabumetone, etodolac, flurbiprofen, diclofenac sodium, piroxicam, betamethasone, dexamethasone, morphine, buprenorphine, diflunisal and acetylsalicylic acid.

8. Liquid galenic formulation according to claim 2, characterized in that antifungal agents are selected from a group consisting of econazole nitrate, miconazole nitrate, clotrimazole, nystatin and ketoconazole.

9. Liquid galenic formulation according to claim 2, characterized in that coronary vasodilators are selected from a group containing isosorbide mono- or dinitrate, nifedipine, nicardipine hydrochloride, nimodipine, nitrendipine, felodipine, amlodipine besilate, isradipine and nicorandil.

10. Liquid galenic formulation according to claim 2, characterized in that antiviral agents are selected from a group consisting of acyclovir, ganciclovir, idoxuridine, interferon and adenosine arabinoside.

11. Liquid galenic formulation according to claim 2, characterized in that miotics and anticholinergics are selected from a group consisting of pilocarpine, epinephrine, physostigmine and neostigmine.

12. Liquid galenic formulation according to claim 2, characterized in that mydriatic agents are selected from a group consisting of atropine, scopolamine and ephedrine.

13. Liquid galenic formulation according to claim 2, characterized in that antiglaucoma agents are selected from a group consisting of timolol maleate, R-timolol and a combination of timolol or R-timolol with pilocarpine.

14. Liquid galenic formulation according to claim 2, characterized in that antihistaminics are selected from a group consisting of disodium chromoglykate, oxatomide, nedocromil, azelastine hydrochloride and ketotifen fumarate.

15. Liquid galenic formulation according to claim 2, characterized in that biogenic peptides are selected from a group consisting of calcitonin, insulin, elcatonin, urokinase, TNF (Tumor Necrosis Factor), erythropoietin, interferons, interleukins and tPA (Tissue Plasminogen Activator).

16. Liquid galenic formulation according to claim 2, characterized in that as cosmetic agents benzoyl peroxide and minoxidil are used.

17. Process for preparing a liquid galenic formulation according to claim 2, characterized in that the carbomer is homogeneously dispersed in demineralized water at room temperature, the pH is adjusted with a pharmaceutically acceptable organic or inorganic base to a value between 4 and 8, then the poloxamer is blended into the clear colloidal dispersion, the whole is left to swell, then it is stirred and a homogeneous dispersion of a therapeutically active amount of the active substance in water is blended into the obtained liquid pharmaceutical carrier.

18. Use of the liquid galenic formulation according to claim 2 by topically applying a liquid galenic formulation onto skin, eye or mucous membranes of the body cavities.

19. Use of the liquid galenic formulation according to claim 2 by topically applying a liquid galenic formulation onto the damaged skin of a patient as a coating protection in burns.

20. Liquid galenic formulation according to claim 2 for topical application of a liquid galenic formulation onto skin, eye or mucous membranes of the body cavities, characterized in that it contains one or more UV adsorbents common in the prevention of sunburns.

21. Use of the liquid galenic formulation according to claim 2 as a protective agent against damages caused by the action of UV irradiation.

22. Liquid galenic formulation according to claim 2, characterized in that it contains an effective dose of an anaesthetic for catheterization or cystoscopy in urological examination.

23. Use of the liquid galenic formulation according to claim 2 as an anaesthetic for catheterization or cystoscopy in urological examination.
